# EUROPÄISCHE PATENTSCHRIFT

(11) **EP 1 682 015 B1**
(45) Veröffentlichungstag und Bekanntmachung des Hinweises auf die Patenterteilung: **16.01.2008**
(21) Anmeldenummer: 04797813.5
(22) Anmeldetag: 09.11.2004
(51) Int. Cl.: A61B 17/50, A61B 17/30

(54) **ZECKENZIEHER**
TICK REMOVER
DISPOSITIF POUR RETIRER LES TIQUES

(30) Priorität: 14.11.2003 DE 20317698 U
(43) Veröffentlichungstag der Anmeldung: 26.07.2006
(73) Patentinhaber: Solutions Branding & Design Companies AG, 20357 Hamburg (DE)
(72) Erfinder: GOLTA, Karel, 22359 Hamburg (DE); PRÜSSNER, Holger, 21244 Rosengarten-Klecken (DE); UHLIG, Günter, 21279 Appel, Eversen-Heide (DE)
(74) Vertreter: Meier, Frank
(86) Internationale Anmeldenummer: PCT/EP2004/012779
(87) Internationale Veröffentlichungsnummer: WO 2005/048855

(56) Entgegenhaltungen:
- WO-A-96/38095
- DE-U- 1 794 844
- FR-A- 1 465 021
- US-A- 4 442 837
- US-A- 5 358 297
- US-B1- 6 179 847

## Beschreibung

Die Erfindung betrifft ein Instrument zum Ausziehen von Zecken aus der Haut von Tieren oder Menschen, mit einem länglichen Griffstück zum Greifen des Instruments einerseits, und einem zumindest teilweise abgeflachten, zumindest teilweise einem ersten Schlitz aufweisenden Endstück zum Greifen der Zecke andererseits, welches Endstück in einer im wesentlichen gabelähnlichen Form endet, welche Form mindestens zwei Zinken aufweist, zwischen denen ein Raum zum Greifen einer Zecke gebildet ist.

Derartige Instrumente sind aus dem Stand der Technik bekannt. So zeigt beispielsweise das EP 0821571 B1 bereits ein Instrument zum Rausziehen von Zecken aus der menschlichen oder tierischen Haut. Diese Druckschrift zeigt insbesondere ein Instrument zum Ausziehen von parasitischen Zecken aus der Haut von Tieren oder Menschen mit einem gekrümmten und abgeflachten Endstück, das in einer Gabelform endet, die aus zwei Zinken besteht, zwischen denen ein Raum zum Greifen der Zecke gebildet ist, wobei das Instrument einstückig geformt ist und einerseits ein Aufnahmeteil runden Querschnitts umfasst, das in einem Griff endet und andererseits das gekrümmte und abgeflachte Endstück umfasst, wobei die Gabelform genau rechtwinklig zur Achse des Aufnahmeteils verläuft und das Instrument die Zecke durch eine Drehung um eine Achse auszieht, die rechtwinklig zur Ebene der Haut, in der die Zecke festsitzt, verläuft.

Aufgabe der vorliegenden Erfindung ist es, die aus dem Stand der Technik bekannten eingangs genannten Instrumente zu verbessern.

Diese Aufgabe wird erfindungsgemäß durch ein Instrument gemäß Anspruch 1 gelöst.

Die Vorteile der vorliegenden Erfindung liegen insbesondere darin, dass es aufgrund der vorliegenden Erfindung möglich ist, den Raum zwischen den mindestens zwei Zinken des im wesentlichen gabelförmigen Endbereichs des Instruments zu variieren. Auf diese Weise kann zum einen großen Zecken Rechnung getragen werden, in dem der vorgenannte Raum vor dem Greifen der Zecke vergrößert wird; zum anderen kann nach dem Ansetzen des Instrumentes, insbesondere nach dem Heranfahren der beiden Zinken auf die Seiten der Zecke, die Zecke zwischen die beiden Zinken eingeklemmt werden, indem das Instrument zusammengedrückt wird, d.h. die in dem Instrument ausgebildeten Schlitze zusammengedrückt werden.

Aufgrund der Variabilität des Raums zwischen den Zinken ist somit dank der Erfindung nur ein Instrument für alle verschiedenen Zeckengrößen notwendig.

Die Erfindung umfasst die Erkenntnis, dass aufgrund der Ausbildung eines Schlitzes auch im Griffabschnitt des Instruments der im Endabschnitt vorgesehene Schlitz zusammengedrückt werden kann. Auf diese Weise ist eine sehr leichte Handhabung des Instruments, insbesondere des Zusammendrückens des Endabschnitts möglich, wobei gleichzeitig durch die Positionierung der Bedienerhand im Griffabschnitt des Instruments eine freie Sicht auf den Endabschnitt des Instruments ermöglicht wird. Es kann daher beim Verwenden des Instruments genau abgeschätzt werden, wann der Endabschnitt zusammengedrückt werden muss bzw. wann der Endabschnitt soweit zusammengedrückt worden ist, dass die Zecke herausgezogen werden kann.

Vorteilhaft ist zumindest einer der Schlitze in seinem Querschnitt V-förmig ausgebildet. Bei der Erfindung sind die beiden Schlitze über ein Filmscharnier miteinander verbunden. Auf diese Weise wird eine erhöhte Stabilität des Instruments erreicht.

Bei einem anderen Ausführungsbeispiel der vorliegenden Erfindung weist der Schlitz aus einer Richtung parallel zu dem Schlitz im Endabschnitt gesehen in einem Übergangsbereich zwischen Endabschnitt und Griffabschnitt eine vergrößerte Weite gegenüber benachbarten Abschnitten des Schlitzes oder der Schlitze auf. Auf diese Weise wird eine Vergrößerung der Variationsbreite des Schlitzes im Endabschnitt erreicht.

Bevorzugt ist der Griffabschnitt als Rotationskörper ausgebildet. Dies ermöglicht einen besonderen Vorteil der Erfindung, nämlich das Greifen der Zecke bei gleichzeitigem Drehen des Instruments zum Lösen der Zecke. Denn durch die Rotationssymmetrie ist beim Drehen des Instruments ein Abrollen des Griffstücks auf der Hand möglich, ohne das die Hand umgreifen muss. Dieser Vorteil des Instruments kann ggf. durch einen um das Griffstück gezogenen Gummiring unterstützt werden.

Eine andere bevorzugte Ausführungsform weist ein im Griffabschnitt integriertes Licht auf, mit welchem der Endabschnitt, insbesondere der Schlitz im Endabschnitt beleuchtbar ist.

Bei einer weiter bevorzugten Ausführungsform der Erfindung ist an dem Instrument eine Lupe derart angebracht, dass insbesondere bei einem Blick parallel zum Griffabschnitt auf den Endabschnitt eine Vergrößerung des Endabschnitts gegeben ist. Bei dieser Variante lässt sich der Endabschnitt besser beobachten und somit die Zecke leichter herausziehen.

Weitere bevorzugte Ausführungsformen der Erfindung sind in den Unteransprüchen angegeben.

Im folgenden werden nun Ausführungsbeispiele anhand der begleitenden Zeichnungen erläutert.
- Figur 1: zeigt eine perspektivische Ansicht einer Ausführungsform des erfindungsgemäßen Instruments;
- Figur 2: zeigt eine Seitenansicht der Ausführungsform der Figur 1;
- Figur 3: zeigt eine Vorderansicht der Ausführungsform der Figur 1; und
- Figur 4: zeigt eine schematische Darstellung des Schlitzes der Ausführungsform der Figur 1.

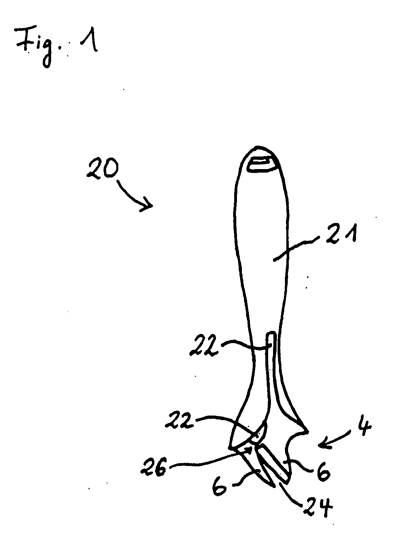

Figur 1 zeigt eine perspektivische Ansicht einer Ausführungsform 20 eines erfindungsgemäßen Instruments zum Ausziehen von Zecken aus der Haut von Tieren oder Menschen. Das Instrument 20 weist einerseits ein längliches Griffstück 21 zum Greifen des Instruments 20 und andererseits ein abgeflachtes Endstück 4 zum Greifen der Zecke auf. Das Endstück 4 endet in einer im wesentlichen gabelähnlichen Form.

Das Endstück 4 weist zwei Zinken 6 auf, zwischen denen ein Raum 10 zum Greifen der Zecke ausgebildet ist.

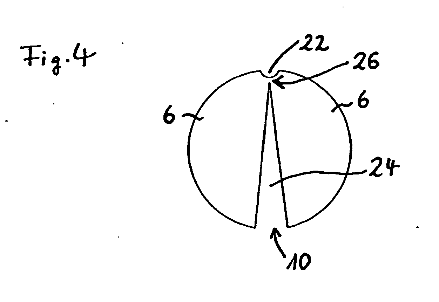

Das Instrument 20 der Figur 1 weist einen ersten im Griffstück 21 ausgebildeten Schlitz 22 und einen zweiten im Endstück 4 ausgebildeten Schlitz 24 auf. Die Schlitze 22 und 24 sind über ein Filmscharnier 26 miteinander verbunden. Der Schlitz 24 öffnet sich zwischen den Zinken 6 des Endstücks 4 V-förmig. Ein Querschnitt in der greifebene des Endstücks 4 durch das Endstück 4 ist in der Figur 4 dargestellt. In dem etwas verlängerten und verdickten Griffstück 21 können ggf. eine den Schlitz 24 beleuchtende und vorzugsweise ein- und ausschaltbare (nicht dargestellte) Lampe eingebracht oder eine (nicht dargestellte) Lupe angebracht werden. Die Oberfläche des Griffstücks 21 ist aus einem rutschfesten Material gebildet.

Bei der Ausführungsform 20 der Figur 1 kann durch Zusammendrücken des Griffstücks 21 der Schlitz 22 und somit über das Filmscharnier 26 auch der Schlitz 24 zusammengedrückt, somit der Raum 10 zwischen den Zinken 6 verengt und schließlich die Zecke auf diese Weise gegriffen werden, nachdem man die beiden zum Zwischenraum 10 hin nach unten hin leicht abgeschrägten Zinken 6 auf beiden Seiten der Zecke unter die in der Haut steckende Zecke geschoben hat.

Da der Schlitz 22 sich nicht durch das gesamte Griffstück 21 erstreckt, ist bei der Ausführungsform 20 kein Ring zum Verhindern eines zu weiten Spreizens des Instruments 20 notwendig.

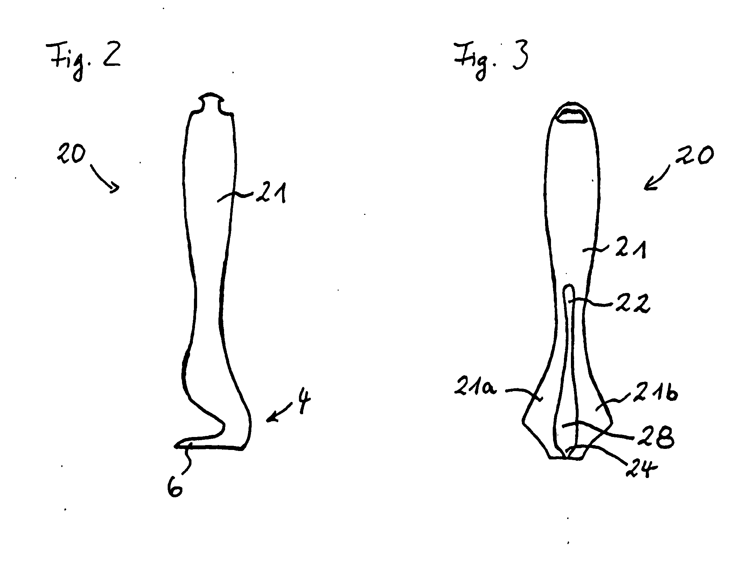

Figur 2 zeigt eine Seitenansicht des Instruments 20 der Figur 1. Figur 3 zeigt eine Frontansicht des Instruments 20 der Figur 1. Aus der Figur 3 ist zu erkennen, dass der Schlitz 22 im Griffstück 21 aus einer Richtung im wesentlichen parallel zu dem ersten Schlitz 24 gesehen eine uneinheitliche, im wesentlichen parallel zur Längserstreckung des Griffstücks 21 variierende und in einem Übergang 28 zwischen Griffstück 21 und Endstück 4 gegenüber zu diesem Übergang 28 benachbarten Abschnitten des Schlitzes 22 vergrößert ist. Auf diese Weise wird das Zusammendrücken der beiden Hälften 21a und 21b des Griffstücks 21 des Instruments 20 erleichtert.

## Patentansprüche

1. Instrument (20) zum Ausziehen von Zecken aus der Haut von Tieren oder Menschen, mit:
einem länglichen Griffstück (21) zum Greifen des Instruments (20) einerseits, und
einem zumindest teilweise abgeflachten, zumindest teilweise einen ersten Schlitz (24) aufweisenden Endstück (4) zum Greifen der Zecke andererseits, welches Endstück (4) in einer im wesentlichen gabelähnlichen Form endet, welche Form mindestens zwei Zinken (6) aufweist, zwischen denen ein Raum (10) zum Greifen einer Zecke gebildet ist, **dadurch gekennzeichnet, dass** das Griffstück (21) einen zweiten Schlitz (22) aufweist, um eine Variation des Raums (10) zwischen den Zinken (6) des Endstücks (4) zu ermöglichen, wobei die Schlitze (22, 24) über ein Filmscharnier miteinander verbunden sind, wobei durch Zusammendrücken des Griffstücks (21) der zweite Schlitz (22) und somit über das Filmscharnier (26) auch der erste Schlitz (24) zusammengedrückt, somit der Raum (10) zwischen den Zinken verengt und schließlich die Zecke auf diese Weise gegriffen werden kann.

2. Instrument (20) nach Anspruch 1,
wobei der erste Schlitz (24) einen im wesentlichen V-förmigen Querschnitt (10) aufweist.

3. Instrument (20) nach einem der vorstehenden Ansprüche,
wobei der zweite Schlitz (22) aus einer Richtung im wesentlichen parallel zu dem ersten Schlitz (24) gesehen eine uneinheitliche Schlitzweite aufweist.

4. Instrument (20) nach dem vorstehenden Anspruch,
wobei die Schlitzweite im wesentlichen parallel zu einer Längserstreckung des Griffstücks (21) variiert.

5. Instrument (20) nach dem vorstehenden Anspruch,
wobei die Schlitzweite zumindest in einem Übergang (28) zwischen Griffstück (21) und Endstück (4) gegenüber zu diesem Übergang (28) benachbarten Abschnitten des Schlitzes (22, 24) vergrößert ist.

6. Instrument (20) nach einem der vorstehenden Ansprüche,
wobei das Griffstück (21) rotationssymmetrisch ist.

7. Instrument (20) nach einem der vorstehenden Ansprüche,
wobei eine Lichtquelle vorgesehen ist, mit der zumindest im wesentlichen das Endstück (4) beleuchtbar ist.

8. Instrument (20) nach einem der vorstehenden Ansprüche,
wobei eine auf das Endstück (4) gerichtete Lupe vorgesehen ist.

## Claims

1. An instrument (20) for removing tricks from the skin of animals or humans, comprising:
an elongate grip part (21) for gripping the instrument (20) at one end, and
an at least partially flattened end piece (4), having at least in part a first slot (24), for gripping the tick at the other end, which end piece (4) ends in a substantially fork-like shape, which shape has at least two prongs (6), between which a space (10) for gripping a tick is formed, **characterised in that** the grip part (21) has a second slot (22) in order to permit varying of the space (10) between the prongs (6) of the end piece (4), the slots (22, 24) being connected together via an integral hinge, the second slot (22) and hence, via the integral hinge (26), also the first slot (24) being able to be pressed together by compressing the grip part (21), thus being able to narrow the space (10) between the prongs and finally grip the tick in this manner.

2. An instrument (20) according to Claim 1,
wherein the first slot (24) has a substantially V-shaped cross-section (10).

3. An instrument (20) according to one of the preceding claims,
wherein the second slot (22), viewed from a direction substantially parallel to the first slot (24), has a non-uniform width.

4. An instrument (20) according to the preceding claim,
wherein the slot width varies substantially parallel to a longitudinal extent of the grip part (21).

5. An instrument (20) according to the preceding claim,
wherein the slot width at least in a transition (28) between the grip part (21) and end piece (4) is enlarged relative to sections of the slot (22, 24) which are adjacent to this transition (28).

6. An instrument (20) according to one of the preceding claims,
wherein the grip part (21) is rotationally symmetrical.

7. An instrument (20) according to one of the preceding claims,
wherein a light source is provided by means of which at least substantially the end piece (4) can be illuminated.

8. An instrument (20) according to one of the preceding claims, wherein a magnifying glass directed at the end piece (4) is provided.

## Revendications

1. Instrument (20) permettant de retirer les tiques de la peau d'animaux ou d'êtres humains, comportant :
une poignée (21) allongée permettant de saisir l'instrument (20) d'un côté, et
un embout (4) au moins partiellement aplati présentant au moins partiellement une première fente (24) pour saisir la tique de l'autre côté, lequel embout (4) se termine par une forme essentiellement semblable à une fourche, laquelle forme présente au moins deux dents (6), entre lesquelles est formé un espace (10) servant à saisir une tique, **caractérisé en ce que** la poignée (21) présente une deuxième fente (22) pour permettre de faire varier l'espace (10) entre les dents (6) de l'embout (4), les fentes (22, 24) étant reliées entre elles par l'intermédiaire d'une charnière à film, la compression de la poignée (21) permettant de comprimer la deuxième fente (22) et ainsi la première fente (24) par l'intermédiaire de la charnière à film (26) pour que l'espace (10) entre les dents puisse être réduit et qu'enfin la tique puisse être saisie de cette manière.

2. Instrument (20) selon la revendication 1,
la première fente (24) présentant une section transversale (10) essentiellement en forme de V.

3. Instrument (20) selon l'une quelconque des revendications précédentes,
la deuxième fente (22) présentant une largeur de fente non uniforme vue depuis une direction essentiellement parallèle par rapport à la première fente (24).

4. Instrument (20) selon la revendication précédente,
la largeur de fente variant essentiellement de manière parallèle par rapport à une extension longitudinale de la poignée (21).

5. Instrument (20) selon la revendication précédente,
la largeur de fente étant agrandie au moins dans un passage (28) entre la poignée (21) et l'embout (4) par rapport à des sections de la fente (22, 24) adjacentes à ce passage (28).

6. Instrument (20) selon l'une quelconque des revendications précédentes,
la poignée (21) étant à symétrie de révolution.

7. Instrument (20) selon l'une quelconque des revendications précédentes,
une source lumineuse étant prévue, avec laquelle essentiellement au moins l'embout (4) peut être éclairé.

8. Instrument (20) selon l'une quelconque des revendications précédentes,
une loupe dirigée vers l'embout (4) étant prévue.
